# EUROPEAN PATENT APPLICATION

(11) **EP 4 548 874 A1**
(43) Date of publication of application: **07.05.2025**
(21) Application number: 24210311.7
(22) Date of filing: 31.10.2024
(51) Int. Cl.: A61B 90/50, A61B 90/00

(54) **SUPPORT ARM APPARATUS AND MEDICAL DEVICE**

(30) Priority: 31.10.2023 CN 202311442300
(71) Applicant: Nanjing Mindray Bio-Medical Electronics Co., Ltd., Nanjing, Jiangsu 211111 (CN)
(72) Inventor: XU, Xiaotian, Nanjing, 211111 (CN); HE, Qicheng, Nanjing, 211111 (CN); SUN, Chao, Nanjing, 211111 (CN); GUO, Bin, Nanjing, 211111 (CN); HUANG, He, Nanjing, 211111 (CN)
(74) Representative: KIPA AB

(57) **Abstract**

A support arm apparatus and a medical device are provided, which include a connection base and a tube including a spring. The connection base includes a first base body, a sleeve connector, and a pull-rod connector which moves towards and away from the sleeve connector during adjustment. The tube includes a sleeve and an elastic part. The elastic part includes a pull-rod and a spring-rod part. A first end of the pull-rod is in rotatable connection with the pull-rod connector about a second axis, a second end of the pull-rod is in rotatable connection with the spring-rod part. The spring-rod part provides a balance force to support a weight of the medical apparatus by the support arm apparatus. The sleeve connector has an avoidance structure for avoiding the pull-rod or the pull-rod connector, to enlarge a movement range of the pull-rod connector and/or a rotation range of the pull-rod.

## Description

### TECHNICAL FIELD

The disclosure relates to the technical field of medical devices, and more particularly to a support arm apparatus and a medical device.

### BACKGROUND

Support arm apparatus is a common mechanism in operating rooms used to support and suspend a medical apparatus, such as a surgical light, a display, and an imaging head. Due to variations in types and weights for the surgical light, display, and imaging head, as well as variations in weight or design for the same product, in order to support the medical apparatuses of different weights, the current industry approach is to develop multiple support arm apparatuses. Each support arm apparatus can support medical apparatuses within a certain weight range. For example, some support arm apparatuses can support medical apparatuses weighing between 7kg and 12kg, some support arm apparatuses can support medical apparatuses weighing between 12kg and 18kg, and some support arm apparatuses can support medical apparatuses weighing between 13.5kg and 21kg. Although this implementation method can support medical apparatuses of different weights, the wide variety of types of support arm apparatuses still greatly increases production management costs.

### SUMMARY

In view of this, the embodiments of this disclosure are expected to provide a support arm apparatus and a medical device.

A first aspect of this disclosure proposes a support arm apparatus which is configured to support a medical apparatus, wherein the support arm apparatus includes a connection base and a tube including a spring; wherein the connection base includes a first base body, a sleeve connector, and a pull-rod connector;
the sleeve connector is installed at the first base body, the pull-rod connector is adjustably installed at the first base body, and is capable of moving towards and away from the sleeve connector during adjustment;
the tube includes:
   a sleeve, which includes a first end and a second end, wherein the first end of the sleeve is in rotatable connection with the sleeve connector about a first axis; and
   an elastic part, which includes a pull-rod and a spring-rod part, wherein the pull-rod includes a first end and a second end, the first end of the pull-rod is in rotatable connection with the pull-rod connector about a second axis, the second end of the pull-rod is in rotatable connection with the spring-rod part, wherein the spring-rod part is installed inside the sleeve, and configured to provide a balance force to enable the support arm apparatus to support a weight of the medical apparatus;
   wherein, the sleeve connector is located in a rotation path of the pull-rod;
   the sleeve connector is provided with an avoidance structure, wherein the avoidance structure is capable of avoiding at least a portion of the pull-rod connector, so as to enlarge a range of movement of the pull-rod connector towards the sleeve connector; and/or the avoidance structure is capable of avoiding at least a portion of the pull-rod, so as to enlarge a range of rotation of the pull-rod towards the sleeve connector.

In a preferable embodiment, the second axis is parallel to the first axis.

In a preferable embodiment, the sleeve connector is a shaft element, the avoidance structure includes a first groove which is arranged at the sleeve connector;
when the pull-rod connector is moved to one extreme position which faces the sleeve connector, at least a portion of the pull-rod connector is capable of being accommodated inside the first groove; and/or when the pull-rod is rotated to another extreme position which faces the sleeve connector, at least a portion of the pull-rod is capable of being accommodated inside the first groove.

In a preferable embodiment, the first groove includes a first bottom wall, and two first side walls which are respectively arranged at both ends of the first bottom wall; wherein the first bottom wall is parallel to the first axis, the two first side walls are spaced apart along the first axis, a distance between the two first side walls is not less than a width of a corresponding portion of the pull-rod connector; wherein when the pull-rod connector is moved to the one extreme position which faces the sleeve connector, at least a portion of the pull-rod connector is capable of entering a space which is defined by the first bottom wall and the two first side walls.

In a preferable embodiment, the avoidance structure further includes a second groove which is arranged at the sleeve connector; wherein the second groove includes a second bottom wall, and two second side walls which are respectively arranged at both ends of the second bottom wall, wherein the second bottom wall is parallel to the first axis, the second bottom wall intersects with the first bottom wall to form an obtuse angle therebetween; the two second side walls are spaced apart along the first axis, a distance between the two second side walls is not less than a width of a corresponding portion of the pull-rod; wherein when the pull-rod is rotated to the another extreme position which faces the sleeve connector, at least a portion of the pull-rod is capable of entering a space which is defined by the second bottom wall and the two second side walls.

In a preferable embodiment, the pull-rod connector is moved along a first direction towards and away from the sleeve connector during adjustment, wherein the first direction is perpendicular to the first bottom wall.

In a preferable embodiment, an inclination angle of the second bottom wall enables a corresponding surface of the pull-rod, which surface faces the second bottom wall, to parallel to or contact with the second bottom wall, when the pull-rod is rotated along the rotation path to approach the second bottom wall.

In a preferable embodiment, the first groove includes a first edge, which is located in the rotation path of the pull-rod; the avoidance structure further includes a second groove which is located at the first edge, wherein the second groove penetrates through a straight line where the first edge is located.

In a preferable embodiment, the first groove includes a first bottom wall, and two first side walls which are respectively arranged at both ends of the first bottom wall; wherein the first bottom wall is parallel to the first axis, the two first side walls are spaced apart along the first axis, a distance between the two first side walls is not less than a width of a corresponding portion of the pull-rod; wherein when the pull-rod is rotated to the another extreme position which faces the sleeve connector, at least a portion of the pull-rod is capable of entering a space which is defined by the first bottom wall and the two first side walls.

In a preferable embodiment, the avoidance structure further includes a second groove which is arranged at the sleeve connector; wherein the second groove includes a second bottom wall, and two second side walls which are respectively arranged at both ends of the second bottom wall, wherein the second bottom wall is parallel to the first axis, the second bottom wall intersects with the first bottom wall to form an obtuse angle therebetween; the two second side walls are spaced apart along the first axis, a distance between the two second side walls is not less than a width of a corresponding portion of the pull-rod connector; wherein when the pull-rod connector is moved to the one extreme position which faces the sleeve connector, at least a portion of the pull-rod connector is capable of entering a space which is defined by the second bottom wall and the two second side walls.

In a preferable embodiment, the pull-rod connector is moved along a first direction towards and away from the sleeve connector during adjustment, wherein the first direction is perpendicular to the second bottom wall.

In a preferable embodiment, the pull-rod connector is adjustably installed at the first base body, and is capable of moving towards and away from the sleeve connector in a first range of movement during adjustment; wherein both ends of the first range of movement are extreme positions between which the pull-rod connector is capable of moving relative to the sleeve connector.

In a preferable embodiment, the pull-rod is capable of rotating towards and away from the sleeve connector in a first range of rotation; wherein both ends of the first range of rotation are extreme positions between which the pull-rod is capable of rotating relative to the sleeve connector.

In a preferable embodiment, the sleeve connector is a shaft element, and the avoidance structure includes a side plane which is arranged at the sleeve connector; wherein the side plane is configured to avoid at least a portion of the pull-rod connector during a movement of the pull-rod connector towards the sleeve connector; and/or the side plane is configured to avoid at least a portion of the pull-rod during a rotation of the pull-rod towards the sleeve connector.

In a preferable embodiment, the shaft element is a portion of a cylinder, and the shaft element is cut by a plane, which plane is parallel to the first axis, so as to form the side plane, wherein the side plane extends from a first end of the shaft element to a second end of the shaft element, a cross-section of the cylinder is circular, and a corresponding central angle of the side plane on the cross-section is less than 180 degrees.

In a preferable embodiment, the sleeve connector includes a first shaft segment, a second shaft segment, and a U-shaped segment, wherein the first shaft segment and the second shaft segment are connected with the first base body, the U-shaped segment is connected between the first shaft segment and the second shaft segment; the avoidance structure is formed by the U-shaped segment; or the avoidance structure is formed by the U-shaped segment, the first shaft segment and the second shaft segment together;
when the pull-rod connector is moved to one extreme position which faces the sleeve connector, at least a portion of the pull-rod connector is capable of being accommodated by the avoidance structure; and/or when the pull-rod is rotated to another extreme position which faces the sleeve connector, at least a portion of the pull-rod is capable of being accommodated by the avoidance structure.

In a preferable embodiment, the U-shaped segment forms an opening between the first shaft segment and the second shaft segment, wherein a width of the opening is not less than a width of a corresponding portion of the pull-rod connector.

In a preferable embodiment, the U-shaped segment forms an opening between the first shaft segment and the second shaft segment, wherein a width of the opening is not less than a width of a corresponding portion of the pull-rod.

In a preferable embodiment, the U-shaped segment includes a portion which is parallel to the first axis, wherein a third groove is arranged at said portion which is parallel to the first axis; when the pull-rod is rotated to another extreme position of a first range of rotation, which position faces the sleeve connector, at least a portion of the pull-rod is capable of being accommodated inside the third groove.

In a preferable embodiment, a convex portion is arranged at a side of the pull-rod, which side faces the sleeve connector; wherein when the pull-rod is rotated to another extreme position which faces the sleeve connector, the convex portion is capable of avoiding at least a portion of the sleeve connector, so as to further enlarge the range of rotation of the pull-rod towards the sleeve connector.

In a preferable embodiment, the pull-rod includes a fitting segment, which is bent away from the sleeve connector to form the convex portion.

In a preferable embodiment, the pull-rod connector is provided with a threaded hole, the connection base further includes an adjustment bolt, which is in threaded engagement with the pull-rod connector; the pull-rod connector is capable of being driven to move towards or away from the sleeve connector within a first range of movement by rotating the adjustment bolt.

In a preferable embodiment, the pull-rod connector is further provided with a guide hole; the connection base further includes a guide column, which is installed at the first base body and penetrates through the guide hole, such that the pull-rod connector is only capable of moving towards or away from the sleeve connector.

In a preferable embodiment, the adjustment bolt is located between the guide column and the pull-rod.

In a preferable embodiment, the sleeve connector is non-rotatably installed at the first base body.

In a preferable embodiment, the first base body includes a first side plate, and a second side plate which is opposite to the first side plate; wherein the first side plate is provided with a first installation hole, the second side plate is provided with a second installation hole; the sleeve connector includes a first installation end and a second installation end, the first installation end penetrates through and is arranged inside the first installation hole, the second installation end penetrates through and is arranged inside the second installation hole; wherein a cross-sectional shape of the first installation end is non-circular, a shape of the first installation hole is adapted to the cross-sectional shape of the first installation end, the first side plate is configured to limit a rotation of the first installation end, so as to make the sleeve connector non-rotatable relative to the first base body; and/or
the first base body includes a first side plate, and a second side plate which is opposite to the first side plate; wherein the first side plate is provided with a first installation hole, the second side plate is provided with a second installation hole, the sleeve connector includes a first installation end and a second installation end, the first installation end penetrates through and is arranged inside the first installation hole, the second installation end penetrates through and is arranged inside the second installation hole; wherein a cross-sectional shape of the second installation end is non-circular, a shape of the second installation hole is adapted to the cross-sectional shape of the second installation end, the second side plate is configured to limit a rotation of the second installation end, so as to make the sleeve connector non-rotatable relative to the first base body.

In a preferable embodiment, the cross-sectional shape of one of the first installation end and the second installation end is D-shaped, and the cross-sectional shape of the other of the first installation end and the second installation end is circular.

In a preferable embodiment, the cross-sectional shape of at least one of the first installation end and the second installation end is non-circular, and includes an arc segment which is in a rotational fit with the sleeve.

In a preferable embodiment, the sleeve includes: a sleeve body, which includes a first end and a second end, wherein the first end of the sleeve body faces the connection base; two first ear portions, which are located at the first end of the sleeve body, wherein one first ear portion is in rotatable connection with the first installation end, and the other first ear portion is in rotatable connection with the second installation end.

In a preferable embodiment, the first side plate and the second side plate are located between the two first ear portions.

In a preferable embodiment, the support arm apparatus further includes an installation base and a rod element, wherein the second end of the sleeve is in rotatable connection with the installation base about a third axis, the installation base is configured to connect the medical apparatus; the rod element includes a first end and a second end, the first end of the rod element is in rotatable connection with the connection base about a fourth axis, and the second end of the rod element is in rotatable connection with the installation base about a fifth axis; wherein the sleeve, the rod element, the connection base, and the installation base are combined to form a parallelogram mechanism having a four-bar linkage .

In a preferable embodiment, the rod element is provided with a groove for cable management.

In a preferable embodiment, the sleeve is provided with a portion for limiting position; the spring-rod part includes the spring, a first slider, and a spring-rod; the spring includes a first end and a second end, the first end of the spring faces the connection base and abuts against the portion for limiting position, the spring-rod penetrates through the spring, the pull-rod is in rotatable connection with the spring-rod, the first slider is connected with the spring-rod and abuts against the second end of the spring.

In a preferable embodiment, the portion for limiting position is located on an inner side wall of the sleeve, the spring is located inside the sleeve, and the first slider is capable of sliding inside the sleeve and abutting against the inner side wall of the sleeve.

In a preferable embodiment, the spring-rod part further includes a second slider, which is located between the second end of the pull-rod and the portion for limiting position, and is connected with the spring-rod; wherein the second slider is capable of sliding inside the sleeve and abutting against the inner side wall of the sleeve.

A second aspect of this disclosure proposes a support arm apparatus which is configured to support a medical apparatus, wherein the support arm apparatus includes a connection base and a tube including a spring; wherein the connection base includes a first base body, a sleeve connector, and a pull-rod connector; the sleeve connector and the pull-rod connector are installed at the first base body;
the tube includes:
a sleeve, which includes a first end and a second end, wherein the first end of the sleeve is in rotatable connection with the sleeve connector about a first axis; and
an elastic part, which includes a pull-rod and a spring-rod part, wherein the pull-rod includes a first end and a second end, the first end of the pull-rod is in rotatable connection with the pull-rod connector about a second axis, the second end of the pull-rod is in rotatable connection with the spring-rod part, wherein the spring-rod part is installed inside the sleeve, and configured to provide a balance force to enable the support arm apparatus to support a weight of the medical apparatus;
wherein, the sleeve connector is located in a rotation path of the pull-rod;
the sleeve connector is provided with an avoidance structure; the avoidance structure is capable of avoiding at least a portion of the pull-rod, so as to enlarge a range of rotation of the pull-rod towards the sleeve connector, during said rotation of the pull-rod towards the sleeve connector.

In a preferable embodiment, the sleeve connector is a shaft element, the avoidance structure includes a groove which is arranged at the sleeve connector; at least a portion of the pull-rod connector is capable of being accommodated inside the groove, during said rotation of the pull-rod towards the sleeve connector.

In a preferable embodiment, the sleeve connector is a shaft element, the avoidance structure includes a side plane which is arranged at the sleeve connector and parallel to the first axis, wherein the side plane extends from a first end of the shaft element to a second end of the shaft element; wherein the side plane is configured to avoid at least a portion of the pull-rod connector, during said rotation of the pull-rod towards the sleeve connector.

In a preferable embodiment, the sleeve connector includes a first shaft segment, a second shaft segment, and a U-shaped segment, wherein the first shaft segment and the second shaft segment are connected with the first base body, the U-shaped segment is connected between the first shaft segment and the second shaft segment; the avoidance structure is formed by the U-shaped segment; or the avoidance structure is formed by the U-shaped segment, the first shaft segment and the second shaft segment together.

In a preferable embodiment, a convex portion is arranged at a side of the pull-rod, which side faces the sleeve connector; wherein when the pull-rod is rotated to an extreme position of a first range of rotation, which position faces the sleeve connector, the convex portion is capable of avoiding at least a portion of the sleeve connector, so as to further enlarge the range of rotation of the pull-rod toward the sleeve connector.

In a preferable embodiment, the pull-rod includes a fitting segment, which is bent away from the sleeve connector to form the convex portion.

A third aspect of this disclosure proposes a medical device, including:
a medical apparatus; and
the above-mentioned support arm apparatus;
wherein the medical apparatus is installed at the second end of the sleeve.

In a preferable embodiment, the medical apparatus is at least one of a surgical light, a display, a control box, and an imaging head.

From the above technical solution, it can be seen that, in the support arm apparatus proposed in the first aspect of this disclosure, firstly, a pull-rod connector is arranged to be movable towards or away from a sleeve connector along a first direction, such that the support arm apparatus is capable of having different balance forces, which is capable of balancing and supporting medical apparatuses of different weights, through adjusting a position of the pull-rod connector relative to the sleeve connector. Secondly, an avoidance structure, which is installed at the sleeve connector, is capable of avoiding at least a portion of the pull-rod connector and/or at least a portion of a pull-rod. When the avoidance structure is configured to avoid at least a portion of the pull-rod connector, the avoidance structure provides a greater adjustment space for the pull-rod connector in a direction towards the sleeve connector, which allows the support arm apparatus to be used to balance and support a medical apparatus with a lighter weight. That is to say, the support arm apparatus proposed in this embodiment can not only be applied to support medical apparatuses of different weights, but also to enlarge a range of support load of the support arm apparatus as a whole. Users do not need to purchase different support arm apparatuses for medical apparatuses of different weights, saving costs for customers. When the avoidance structure is configured to avoid at least a portion of the pull-rod, the avoidance structure allows the pull-rod to have a larger angle of rotation towards the sleeve connector, that is, the tube has a larger depression angle of rotation, which is capable of facilitating adjustment, installation, and disassembly of medical apparatus by a user.

### BRIEF DESCRIPTION OF THE DRAWINGS

In order to clearly illustrate technical solutions of embodiments of this disclosure, a brief introduction is given to accompanying drawings required for description of the embodiments. It is obvious that the accompanying drawings described below are some embodiments of this disclosure. For those skilled in the art, other drawings can be obtained from these drawings without creative labour.
FIG. 1 is a structural diagram of a support arm apparatus in a first state proposed in an embodiment of this disclosure.
FIG. 2 is a sectional view of a partial structure of a support arm apparatus proposed in an embodiment of this disclosure.
FIG. 3 is a schematic diagram of a sleeve connector, a pull-rod connector, and a pull-rod proposed in an embodiment of this disclosure.
FIG. 4 is a structural diagram of a sleeve connector proposed in an embodiment of this disclosure.
FIG. 5 is a schematic diagram of a pull-rod connector proposed in an embodiment of this disclosure, in which the pull-rod connector is moved to a highest point.
FIG. 6 is a schematic diagram of a pull-rod connector proposed in an embodiment of this disclosure, in which the pull-rod connector is moved to a lowest point.
FIG. 7 is a structural diagram of a sleeve connector proposed in another embodiment of this disclosure.
FIG. 8 is a structural diagram of a sleeve connector proposed in another embodiment of this disclosure.
FIG. 9 is a structural diagram of a sleeve connector proposed in another embodiment of this disclosure.
FIG. 10 is a structural diagram of a sleeve connector proposed in another embodiment of this disclosure.
FIG. 11 is a structural diagram of a sleeve connector proposed in another embodiment of this disclosure.
FIG. 12 is a structural diagram of a sleeve connector proposed in another embodiment of this disclosure.
FIG. 13 is a structural diagram of a sleeve connector proposed in another embodiment of this disclosure.
FIG. 14 is a sectional view of a partial structure of a support arm apparatus proposed in another embodiment of this disclosure.
FIG. 15 is a sectional view of a partial structure of a support arm apparatus proposed in another embodiment of this disclosure.
FIG. 16 is a structural diagram of a connection base proposed in an embodiment of this disclosure from a first perspective.
FIG. 17 is a structural diagram of a connection base proposed in an embodiment of this disclosure from a second perspective.
FIG. 18 is a structural diagram of a sleeve proposed in an embodiment of this disclosure.
FIG. 19 is an assembly diagram of a sleeve connector, and a limitation element proposed in another embodiment of this disclosure.
FIG. 20 is a structural diagram of a support arm apparatus in a second state proposed in an embodiment of this disclosure.
FIG. 21 is a structural diagram of an installation base proposed in an embodiment of this disclosure.
FIG. 22 is a partial structural diagram of a support arm apparatus proposed in an embodiment of this disclosure.

### DESCRIPTION OF THE PREFERRED EMBODIMENTS

Technical solutions in embodiments of this disclosure are clearly and completely described in conjunction with the accompanying drawings as follows. Obviously, the described embodiments are some of the embodiments of this disclosure, not all of them. Based on the embodiments of this disclosure, all other embodiments obtained by ordinary skilled in the art without creative labor are within the scope of protection of this disclosure.

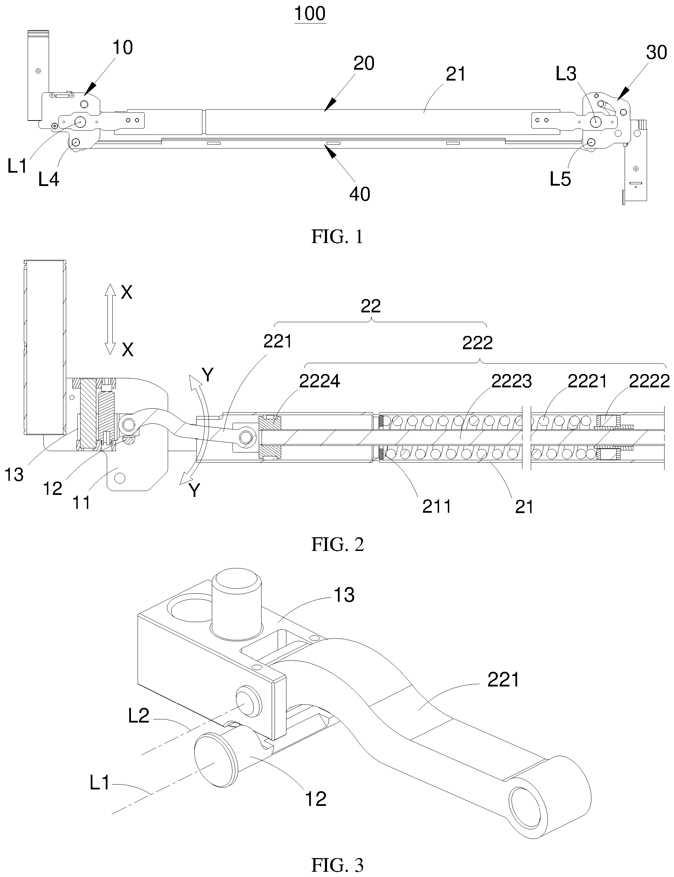

As shown in FIG. 1 to 4, an embodiment of this disclosure proposes a support arm apparatus 100 which is configured to support a medical apparatus. The proposed support arm apparatus 100 includes a connection base 10 and a tube 20 which includes a spring. The connection base 10 includes a first base body 11, a sleeve connector 12, and a pull-rod connector 13. The sleeve connector 12 is non-rotatably installed at the first base body 11, and the pull-rod connector 13 is adjustably installed at the first base body 11 and is capable of moving towards and away from the sleeve connector 12 along a first direction X within a first range of movement, during adjustment.

Both ends of the first range of movement are extreme positions between which the pull-rod connector 13 is capable of moving relative to the sleeve connector 12. For example, when the pull-rod connector 13 is moved towards the sleeve connector 12 to an end of the first range of movement, the pull-rod connector 13 cannot be moved towards the sleeve connector 12 any further. Conversely, when the pull-rod connector 13 is moved away from the sleeve connector 12 to the other end of the first range of movement, the pull-rod connector 13 cannot be moved away from the sleeve connector 12 any further.

The tube 20 includes a sleeve 21 and an elastic part 22. The sleeve 21 includes a first end and a second end, wherein the first end of the sleeve 21 is in rotatable connection with the sleeve connector 12 about a first axis L1. The elastic part 22 includes a pull-rod 221 and a spring-rod part 222. The pull-rod 221 includes a first end and a second end. The first end of the pull-rod 221 is in rotatable connection with the pull-rod connector 13 about a second axis L2. The pull-rod 221 is capable of rotating towards and away from the sleeve connector 12 in a second direction Y within a first range of rotation.

Both ends of the first range of rotation are extreme positions between which the pull-rod 221 is capable of rotating relative to the sleeve connector 12. For example, when the pull-rod 221 is rotated towards the sleeve connector 12 to an end of the first range of rotation, the pull-rod 221 cannot be rotated towards the sleeve connector 12 any further. Conversely, when the pull-rod 221 is rotated away from the sleeve connector 12 to the other end of the first range of rotation, the pull-rod 221 cannot be rotated away from the sleeve connector 12 any further.

The second end of the pull-rod 221 is in rotatable connection with the spring-rod part 222, which is installed inside the sleeve 21. The spring-rod part 222 is configured to provide a balance force to enable the support arm apparatus 100 to support a weight of the medical apparatus. The sleeve connector 12 is located in a rotation path of the pull-rod 221. The sleeve connector 12 is provided with an avoidance structure A. When the pull-rod connector 13 is moved to an extreme position of the first range of movement, which position faces the sleeve connector 12, the avoidance structure A is capable of avoiding at least a portion of the pull-rod connector 13, so as to enlarge the range of movement of the pull-rod connector 13 towards the sleeve connector 12.

In one embodiment, weights of the sleeve connector 12, as well as of the medical apparatus which is suspended by the spring-rod part 222 and the second end of the sleeve 21 (if there is a suspended medical apparatus), jointly define the first range of rotation of the pull-rod 221. The sleeve connector 12 defines an extreme position of the rotation of the pull-rod 221 towards the sleeve connector 12, and the weight of the medical apparatus, which is suspended by the spring-rod part 222 and the second end of the sleeve 21, defines an extreme position of the rotation of the pull-rod 221 away from the sleeve connector 12.

In one embodiment, the support arm apparatus 100 further includes an installation base 30 which is connected with the second end of the sleeve 21. Taking application of the support arm apparatus 100 in an operating room as an example, in one usage scenario, the support arm apparatus 100 is directly or indirectly connected with a ceiling of the operating room through its connection base 10, and a second end of the support arm apparatus 100 is connected with a medical apparatus, such as a surgical light, display, control box, and imaging head, through its installation base 30. The weight of the medical apparatus, such as a surgical light, display, control box, and imaging head, varies. Users can adjust a position of the pull-rod connector 13 relative to the sleeve connector 12 according to the weight of the medical apparatus to be installed, so as to enable the tube 20 to have a support force that is compatible with the medical apparatus. Then, the medical apparatus can be installed at the installation base 30. Of course, the medical apparatus can also be installed at the installation base 30 first, and then the position of the pull-rod connector 13 relative to the sleeve connector 12 can be adjusted according to the weight of the medical apparatus, so as to enable the tube 20 to have a support force that is compatible with the medical apparatus.

It should be noted that the shorter a distance between the first axis L1 and the second axis L2, the smaller a balance force that the spring-rod part 222 can form, such that the support arm apparatus 100 can support a medical apparatus with a lighter weight. In the above embodiment, by arranging the avoidance structure A at the sleeve connector 12, the pull-rod connection 13 can be further moved towards the sleeve connector 12, so that the distance between the first axis L1 and the second axis L2 can be shorter, and the support arm apparatus 100 can support a medical apparatus with a lighter weight.

It should also be noted that the avoidance structure A is not limited to being configured to avoid the pull-rod connector 13. For example, in another embodiment, when the pull-rod 221 is rotated to an extreme position of the first range of rotation, which position faces the sleeve connector 12, the avoidance structure A is capable of avoiding at least a portion of the pull-rod 221, so as to enlarge the range of rotation of the pull-rod 221, which rotation faces the sleeve connector 12. In this embodiment, the pull-rod 221 has a larger angle of rotation, which rotation faces the sleeve connector 12, that is, the tube 20 has a larger depression angle of rotation, which can facilitate adjustment, installation, and disassembly of medical apparatus by users. In another embodiment, the avoidance structure A is not only configured to avoid the pull-rod connector 13, so as to enlarge the range of movement of the pull-rod connector 13 towards the sleeve connector 12, but also to avoid the pull-rod 221, so as to enlarge the range of rotation of the pull-rod 221 towards the sleeve connector 12.

In the support arm apparatus 100 proposed in this embodiment, firstly, a pull-rod connector 13 is arranged to be movable towards or away from a sleeve connector 12 along a first direction X, such that the support arm apparatus 100 is capable of having different balance forces, which can balance and support medical apparatuses of different weights, through adjusting a position of the pull-rod connector 13 relative to the sleeve connector 12. Secondly, an avoidance structure A, which is installed at the sleeve connector 12, is capable of avoiding at least a portion of the pull-rod connector and/or at least a portion of the pull-rod. When the avoidance structure A is configured to avoid at least a portion of the pull-rod connector, the avoidance structure A provides a greater adjustment space for the pull-rod connector in a direction towards the sleeve connector 12, which allows the support arm apparatus to be used to balance and support a medical apparatus with a lighter weight. That is to say, the support arm apparatus 100 proposed in this embodiment can not only be applied to support medical apparatuses of different weights, but also to enlarge a range of support load of the support arm apparatus 100 as a whole. Users do not need to purchase different support arm apparatuses 100 for medical apparatuses of different weights, saving costs for customers. When the avoidance structure A is configured to avoid at least a portion of the pull-rod, the avoidance structure A allows the pull-rod 221 to have a larger angle of rotation towards the sleeve connector 12, that is, the tube 23 has a larger depression angle of rotation, which can facilitate adjustment, installation, and disassembly of medical apparatus by user.

As shown in FIG. 2, in one embodiment, a portion for limiting position 211 is provided at the sleeve 21, the spring-rod part 222 includes a spring 2221, a first slider 2222, and a spring-rod 2223. The spring 2221 includes a first end and a second end, wherein the first end of the spring 2221 faces the connection base 10 and abuts against the portion for limiting position 211. The spring-rod 2223 penetrates through the spring 2221, and the pull-rod 221 is in rotatable connection with the spring-rod 2223. The first slider 2222 is connected with the spring-rod 2223 and abuts against the second end of the spring 2221. It should be noted that the first slider 2222 is connected with the spring-rod 2223, means that the first slider 2222 can be directly connected with the spring-rod 2223, or the first slider 2222 can be flexibly sleeved around the spring-rod 2223. A preload bolt is arranged at a side of the first slider 2222, which side faces away from the spring 2221, and the preload bolt is in thread engagement with the spring-rod 2223. The preload bolt abuts against the first slider 2222, and the first slider 2222 remains relatively stationary relative to the spring-rod 2223 under a clamping of the preload bolt and the spring 2221. The specific design can be determined according to actual needs.

Generally, after the support arm apparatus 100 leaves the factory, the first slider 2222 is set to have a certain amount of preset compression on the spring 2221. The spring 2221 generates a reaction force due to said compression, so as to enable the pull-rod 221 to exert a pulling force to the pull-rod connector 13. A vertical component of this pulling force is exerted to the sleeve 21 through the first slider 2222, so as to support the medical apparatus which is installed at the installation base 30. When the user needs to install a heavier medical device at the installation base 30, the user adjusts the pull-rod connector 13 to move away from the sleeve connector 12. The pull-rod 221 pulls the spring-rod 2223 to move to the connection base 10, and the spring-rod 2223 drives the first slider 2222 to move to the connection base 10. The moved first slider 2222 further compresses the spring 2221, and the reaction force provided by the spring 2221 increases the pulling force of the pull-rod 221 towards the pull-rod connector 13. A vertical component of the increased pulling force increases, thereby supporting a heavier medical device. On the contrary, when the user adjusts the pull-rod connector 13 to move towards the sleeve connector 12, the weight of the medical apparatus that the support arm apparatus 100 can support decreases.

As shown in FIG. 2, in one embodiment, the portion for limiting position 211 is arranged on an inner side wall of the sleeve 21, the spring 2221 is arranged inside the sleeve 21, and the first slider 2222 is capable of sliding inside the sleeve 21 and abutting against the inner side wall of the sleeve 21. In this embodiment, by setting the first slider 2222 to abut against the inner side wall of the sleeve 21, the first slider 2222 can serve as a positioning tool for the spring-rod 2223. It should be noted that it is also possible to set the first slider 2222 to not abut against the inner side wall of the sleeve 21, as long as the first slider 2222 can abut against the spring 2221, which can be determined according to actual design requirements.

As shown in FIG. 2, in one embodiment, the spring-rod part 222 further includes a second slider 2224, which is located between the second end of the pull-rod 221 and the portion for limiting position 211 and connected with the spring-rod 2223. The second slider 2224 is capable of sliding inside the sleeve 21 and abutting against the inner side wall of the sleeve 21. Similarly, by setting the second slider 2224 to abut against the inner side wall of the sleeve 21, the second slider 2224 can serve as a positioning tool for the spring-rod 2223. It should be noted that the first slider 2222, in conjunction with the second slider 2224, can position the spring-rod 2223 at a central axis of the sleeve 21, meaning that the spring-rod 2223 can only move backward and forward along the central axis of the sleeve 21.

As shown in FIG. 3, in one embodiment, the second axis L2 is parallel to the first axis L1. Of course, in other embodiments, the second axis L2 can also be non-parallel to the first axis L1, depending on actual design requirements.

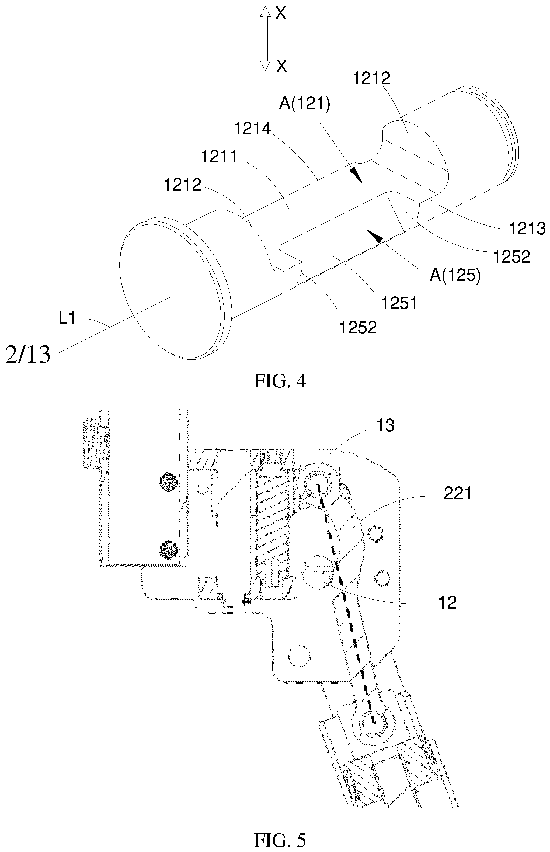

As shown in FIG. 4, in one embodiment, the sleeve connector 12 is a shaft element, and the avoidance structure A includes a first groove 121 which is arranged at the sleeve connector 12. When the pull-rod connector 13 is moved to the extreme position of the first range of movement, which position faces the sleeve connector 12, at least a portion of the pull-rod connector 13 can be accommodated inside the first groove 121. That is, in this embodiment, at least a portion of the pull-rod connector 13 is accommodated by the sleeve connector 12 through the first groove 121, which allows the pull-rod connector 13 to move towards the sleeve connector 12 by a greater distance. In the illustrated embodiment, the first groove 121 is located in a middle of the sleeve connector 12. Of course, the first groove 121 is not limited to being in the middle of the sleeve connector 12. In other embodiments, the first groove 121 can also be in other positions of the sleeve connector 12, depending on actual design requirements.

It should be noted that the first groove 121 is not limited to being accommodated inside at least a portion of the pull-rod connector 13. In another embodiment, when the pull-rod 221 is rotated to an extreme position of the first range of rotation, which position faces the sleeve connector 12, at least a portion of the pull-rod 221 is capable of being accommodated inside the first groove 121. That is, in this embodiment, at least a portion of the pull-rod 221 is accommodated by the sleeve connector 12 through the first groove 121, which allows the pull-rod 211 to rotate towards the sleeve connector 12 by a larger angle.

As shown in FIG. 4, in one embodiment, the first groove 121 includes a first bottom wall 1211, and two first side walls 1212 which are respectively located at both ends of the first bottom wall 1211. The first bottom wall 1211 is arranged parallel to the first axis L1, and the two first side walls 1212 are spaced apart along the first axis L1. A distance between the two first side walls 1212 is not less than a width of a corresponding portion of the pull-rod connector 13. When the pull-rod connector 13 is moved to an extreme position of the first range of movement, which position faces the sleeve connector 12, at least a portion of the pull-rod connector 13 is capable of entering a space which is defined by the first bottom wall 1211 and the two first side walls 1212.

As shown in FIG. 4, in one embodiment, the pull-rod connector 13 moves along the first direction X towards and away from the sleeve connector 12 during adjustment, and the first direction X is perpendicular to the first bottom wall 1211. It should be noted that the first bottom wall 1211 is not limited to being perpendicular to the first direction X. In other embodiments, the first bottom wall 1211 can also have an acute or obtuse angle with respect to the first direction X, as long as a highest point of the first bottom wall 1211 is lower than a highest point of a cylinder where the sleeve connector 12 is located, in the first direction X, so that the sleeve connector 12 is capable of avoiding the pull-rod connector 13.

As shown in FIG. 4, in one embodiment, the avoidance structure A further includes a second groove 125 which is arranged at the sleeve connector 12. The second groove 125 includes a second bottom wall 1251, and two second side walls 1252 which are respectively located at both ends of the second bottom wall 1251. The second bottom wall 1251 is arranged parallel to the first axis L1, and the second bottom wall 1251 intersects with the first bottom wall 1211 to form an obtuse angle therebetween. The two second side walls 1252 are spaced apart along the first axis L1. A distance between the two second side walls 1252 is not less than a width of a corresponding portion of the pull-rod 221. When the pull-rod 221 is rotated to an extreme position of the first range of rotation, which position faces the sleeve connector, at least a portion of the pull-rod 221 is capable of entering a space which is defined by the second bottom wall 1251 and the two second side walls 1252.

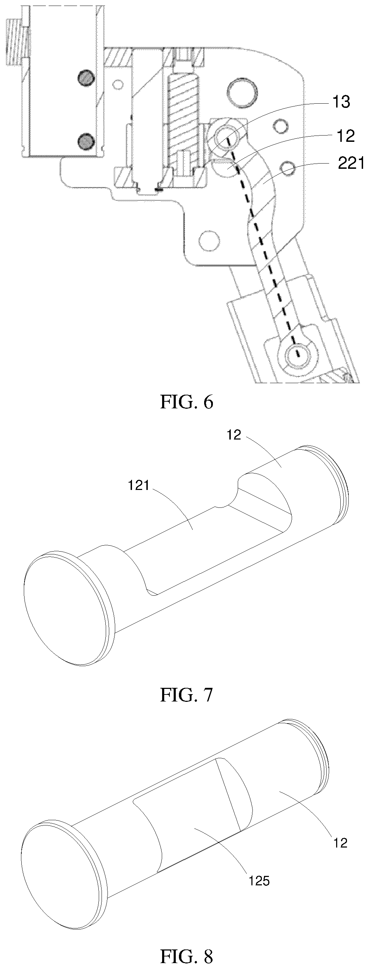

As shown in FIG. 6, in one embodiment, an inclination angle of the second bottom wall 1251 enables a corresponding surface of the pull-rod 221, which surface faces the second bottom wall 1251, to parallel to or contact with the second bottom wall 1251, when the pull-rod 221 is rotated along a rotation path to approach the second bottom wall 1251.

As shown in FIG. 4, in one embodiment, the first groove 121 includes a first edge 1213 and an opposite second edge 1214, wherein the first edge 1213 is closer to the installation base 30 than the second edge 1214. The first edge 1213 is located in the rotation path of the pull-rod 221. The avoidance structure A also includes a second groove 125, which is located at the first edge 1213 of the sleeve connector 12 and penetrates through a straight line where the first edge 1213 is located. The second groove 125 is configured to avoid the pull-rod 221, so as to enlarge the range of rotation of the pull-rod 221 towards the sleeve connector 12. In this implementation, the pull-rod 221 has a larger angle of rotation towards the sleeve connector 12, which means that the tube 20 has a larger depression angle of rotation, making it convenient for users to adjust, install, and disassemble the medical apparatus.

In the above embodiment, the avoidance structure A includes both a first groove 121 and a second groove 125. The first groove 121 is configured to avoid the pull-rod connector 13, and the second groove 125 is configured to avoid the pull-rod 221, so that the pull-rod connector 13 has a larger adjustment space in a direction towards the sleeve connector 12, and the support arm apparatus 100 can be used to balance and support a medical apparatus with a lighter weight. Furthermore, as the pull-rod 221 is allowed to have a larger angle of rotation towards the sleeve connector 12, that is, the tube 20 is allowed to have a larger depression angle of rotation, such that adjustment, installation, and disassembly of the medical apparatus by user is facilitated. Specifically, as shown in FIG. 5 and 6, by setting the first groove 121 and the second groove 125, the pull-rod connector 13 can be partially inserted into the first groove 121 when it moves towards the sleeve connector 12 to an extreme position, achieving a larger range of movement. Moreover, as can be seen from FIG. 6, the second groove 125 allows the pull-rod 221 to be inclined further downward, thereby giving the tube 20 a larger rotational depression angle.

It should be noted that although both the first groove 121 and the second groove 125 are provided in the embodiments of this disclosure, only the first groove 121 may be provided, as shown in FIG. 7, or only the second groove 125 may be provided, as shown in FIG. 8. It should be noted that when a highest point of the second groove 125 is lower than a highest point of the cylinder where the installation end is located in the first direction X, avoidance of the pull-rod 221 and the pull-rod connector 13 can be realized.

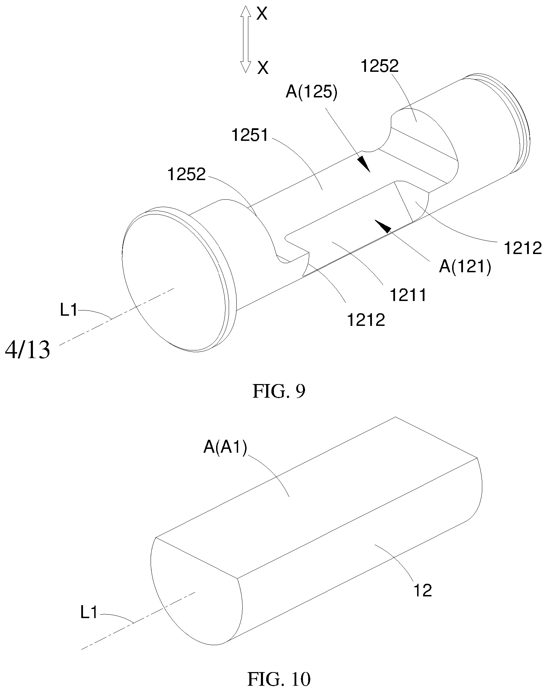

As shown in FIG. 9, in one embodiment, the avoidance structure A includes a first groove 121, wherein the first groove 121 includes a first bottom wall 1211, and two first side walls 1212 which are respectively located at both ends of the first bottom wall 1211. The first bottom wall 1211 is parallel to the first axis L1, and the two first side walls 1212 are spaced apart along the first axis L1. A distance between the two first side walls 1212 is not less than a width of a corresponding portion of the pull-rod 221. When the pull-rod 221 is rotated to an extreme position of the first range of rotation, which position faces the sleeve connector 12, at least a portion of the pull-rod 221 is capable of entering a space which is defined by the first bottom wall 1211 and the two first side walls 1212.

As shown in FIG. 9, in one embodiment, the avoidance structure A further includes a second groove 125 which is arranged at the sleeve connector 12. The second groove 125 includes a second bottom wall 1251 and two second side walls 1252 which are respectively located at both ends of the second bottom wall 1251. The second bottom wall 1251 is arranged parallel to the first axis L1, and the second bottom wall 1251 intersects with the first bottom wall 1211 and forms an obtuse angle therebetween. The two second side walls 1252 are spaced apart along the first axis L1. A distance between the two second side walls 1252 is not less than a width of a corresponding portion of the pull-rod connector 13. When the pull-rod connector 13 is moved to an extreme position of the first range of movement, which position faces the sleeve connector, at least a portion of the pull-rod connector 13 is capable of entering a space which is defined by the second bottom wall 1251 and the two second side walls 1252.

As shown in FIG. 9, in one embodiment, the pull-rod connector 13 moves along the first direction X towards and away from the sleeve connector 12 during adjustment, and the first direction X is perpendicular to the second bottom wall 1251. It should be noted that the second bottom wall 1251 is not limited to being perpendicular to the first direction X. In other embodiments, the second bottom wall 1251 can also have an acute or obtuse angle with respect to the first direction X, as long as a highest point of the second bottom wall 1251 is lower than a highest point of a cylinder where the sleeve connector 12 is located in the first direction X, so that the sleeve connector 12 is capable of avoiding the pull-rod connector 13.

It should be noted that the sleeve connector 12 is not limited to the above embodiments. For example, in another embodiment, as shown in FIG. 10, the sleeve connector 12 is a shaft element, and the avoidance structure A includes a side plane A1 which is arranged at the sleeve connector 12. During the movement of the pull-rod connector 13 towards the sleeve connector 12, the side plane A1 is configured to avoid at least a portion of the pull-rod connector 12.

As shown in FIG. 10, in one embodiment, during the rotation of the pull-rod 221 towards the sleeve connector 12, the side plane A1 is configured to avoid at least a portion of the pull-rod 221.

As shown in FIG. 10, in one embodiment, the shaft element is a portion of a cylinder and is cut by a plane, which is parallel to the first axis L1, so as to form a side plane A1. The side plane A1 extends from a first end of the shaft element to a second end of the shaft element. A cross-section of the cylinder is circular, and a corresponding central angle of the side plane A1 on the cross-section is less than 180 degrees.

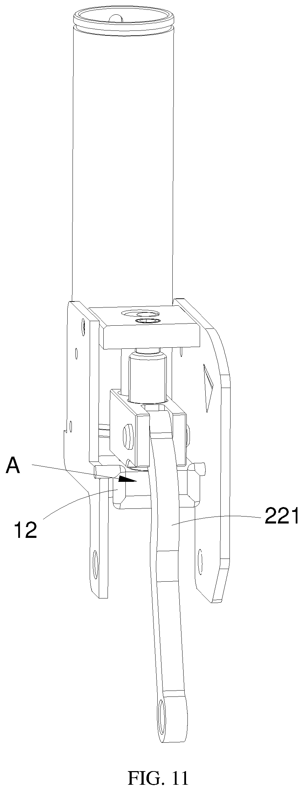

It should be noted that the sleeve connector 12 is not limited to a shaft element, and the avoidance structure A is not limited to the above-mentioned method. For example, in another embodiment, as shown in FIG. 11, 12, 14, and 15, the sleeve connector 12 includes a first shaft segment 122, a second shaft segment 123, and a U-shaped segment 124. The first shaft segment 122 and the second shaft segment 123 are connected with the first base body 11 and capable of extending along the first axis L1. The U-shaped segment 124 is connected between the first shaft segment 122 and the second shaft segment 123. In the illustrated embodiment, a space which is enclosed by the U-shaped segment 124, forms the avoidance structure A. When the pull-rod connector 13 is moved to the extreme position of the first range of movement, which position faces the sleeve connector 12, at least a portion of the pull-rod connector 13 is capable of being accommodated by the avoidance structure A. It should be noted that in this embodiment, according to design requirements, by setting a depth of convex of the U-shaped segment 124, the pull-rod connector 13 can move towards the sleeve connector 12 to almost coincide with the second axis L2 and the first axis L1.

It should also be noted that the avoidance structure A is not limited to avoiding the pull-rod connector 13. For example, in another embodiment, when the pull-rod 221 is rotated to the extreme position of the first range of rotation, which position faces the sleeve connector 12, at least a portion of the pull-rod 221 is capable of being accommodated by the avoidance structure A.

It should be noted that the avoidance structure A is not limited to being formed solely by the U-shaped segment 124. For example, in other embodiments, the avoidance structure A can be formed by the U-shaped segment 124, the first shaft segment 122 and the second shaft segment 123 together, that is, the U-shaped segment only includes a portion which is located below the first shaft segment 122 and the second shaft segment 123.

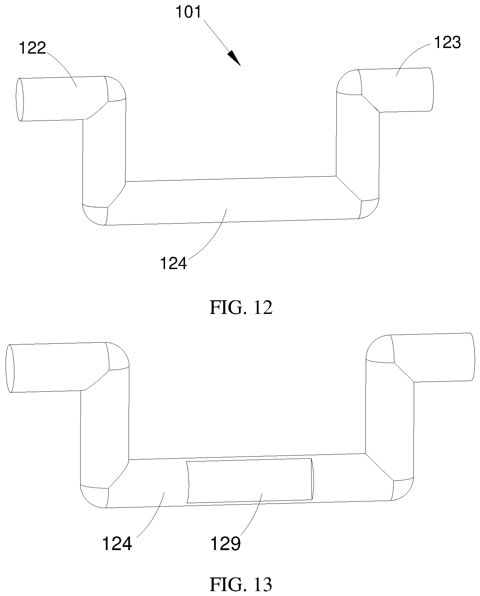

As shown in FIG. 12, in one embodiment, the U-shaped segment 124 forms an opening 101 between the first shaft segment 122 and the second shaft segment 123, and a width of the opening 101 is not less than a width of a corresponding portion of the pull-rod connector 13. When the pull-rod connector 13 is moved towards the sleeve connector 12, it is capable of entering a space which is enclosed by the U-shaped segment 124, through the opening 101. In one embodiment, the width of the opening 101 is not less than a width of a corresponding portion of the pull-rod 221. When the pull-rod 221 is rotated towards the sleeve connector 12, it can enter a space which is enclosed by the U-shaped segment 124, through the opening 101.

As shown in FIG. 13, in one embodiment, the U-shaped segment 124 includes a portion which is parallel to the first axis L1, and a third groove 129 is arranged at the portion which is parallel to the first axis L1. When the pull-rod 221 is rotated to an extreme position of the first range of rotation, which position faces the sleeve connector 12, at least a portion of the pull-rod 221 is capable of being accommodated inside the third groove 129. With this implementation, the angle of rotation of the pull-rod 221 towards the U-shaped segment 124 can be further enlarged, which allows the tube 20 to have a larger depression angle of rotation.

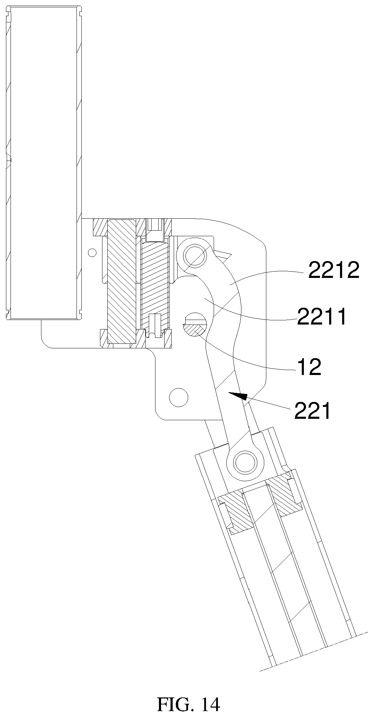

As shown in FIG. 14, in one embodiment, a convex portion 2211 is arranged at a side of the pull-rod 221, which side faces the sleeve connector 12. When the pull-rod 221 is rotated to an extreme position of the first range of rotation, which position faces the sleeve connector 12, the convex portion 2211 is capable of avoiding at least a portion of the sleeve connector 12 to further enlarge the range of rotation of the pull-rod 221 toward the sleeve connector 12. With this implementation, the pull-rod 221 has a larger angle of rotation towards the sleeve connector 12, and the support arm apparatus 100 has a larger depression angle of rotation, making it easier for users to adjust the medical apparatus.

As shown in FIG. 14, in one embodiment, the pull-rod 221 includes a fitting segment 2212, which is bent away from the sleeve connector 12 to form a convex portion 2211. The bent shape of the fitting segment 2212 can be an arc shape, a curved shape, a broken line shape, etc. It should be noted that the convex portion 2211 is not limited to being formed in the above-mentioned manner. For example, in another embodiment, the fitting segment 2212 is a straight segment, and a convex is arranged at a side of the fitting segment 2212, which side faces the sleeve connector 12, so as to form the convex portion 2211.

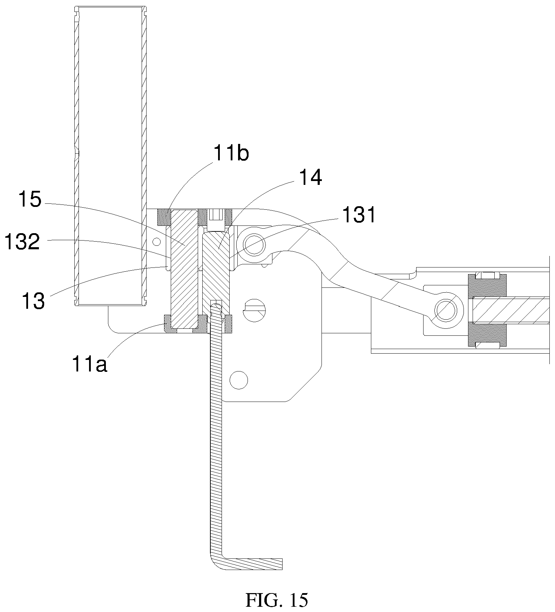

As shown in FIG. 15, in one embodiment, the pull-rod connector 13 is provided with a threaded hole 131, and the connection base 10 further includes an adjustment bolt 14, which is in threaded engagement with the pull-rod connector 13. The pull-rod connector 13 can be driven to move towards or away from the sleeve connector 12 within a first range of movement by rotating the adjustment bolt 14.

In one embodiment, one end of the adjustment bolt 14 is provided with a sink groove. The sink groove can be, but is not limited to, a straight sink groove, a cross sink groove, or a hexagonal sink groove. In this way, when the adjustment bolt 14 needs to be rotated, the operator can insert a straight screwdriver, a cross screwdriver or a hexagonal wrench into the sink groove and rotate it to drive the adjustment bolt 14 to rotate.

As shown in FIG. 15, in one embodiment, the pull-rod connector 13 is also provided with a guide hole 132, and the connection base 10 further includes a guide column 15. The guide column 15 is installed at the first base body 11 and penetrates through the guide hole 132, so that the pull-rod connector 13 can only move towards or away from the sleeve connector 12. In this implementation, the guide column 15 is arranged to guide the pull-rod connector 13, so that the pull-rod connector 13 can move smoothly during the adjustment process.

As shown in FIG. 15, in one embodiment, the first base body 11 includes a bottom connector 11a and a top connector 11b. A bottom end of the guide column 15 is connected with the bottom connector 11a, and a top end of the guide column 15 is connected with the top connector 11b. The bottom connector 11a and the top connector 11b jointly define the first range of movement of the pull-rod connector 13, wherein the bottom connector 11a defines an extreme position of a movement of the pull-rod connector 13 towards the sleeve connector 12, and the top connector 11b defines an extreme position of a movement of the pull-rod connector 13 away from the sleeve connector 12.

It should be noted that the first range of movement of the pull-rod connector 13 is not defined through the scheme of the bottom connector 11a and the top connector 11b mentioned above. For example, in some other embodiments, a convex block can be arranged at both ends of the guide column 15, and the first range of movement of the pull-rod connector 13 can be jointly defined by the two convex blocks. Alternatively, in some other embodiments, the top connector 11b defines an extreme position of a movement of the pull-rod connector 13 away from the sleeve connector 12, and the sleeve connector 12 defines an extreme position of a movement of the pull-rod connector 13 towards the sleeve connector 12. These depend on the actual design requirements.

As shown in FIG. 15, in one embodiment, the adjustment bolt 14 is located between the guide column 15 and the pull-rod 221. It can be understood that the tube 20, due to its own weight and the weight of the medical apparatus, has a tendency to pull the pull-rod connector 13 to rotate downward about the first axis L1, causing the inner side wall of the guide hole 132 to press against the guide column 15, or causing the inner side wall of the threaded hole 131 to press against the adjustment bolt 14. When the inner side wall of the threaded hole 131 presses against the adjustment bolt 14, the operator requires a large operation force during the rotation of the adjustment bolt 14, making adjustment inconvenient. In this embodiment, by setting the adjustment bolt 14 between the guide column 15 and the pull-rod 221, the outer side wall of the guide column 15 shares more pressure, and the outer side wall of the adjustment bolt 14 shares less pressure, making it easier for the operator to rotate the adjustment bolt 14. Of course, it is also possible to set the guide column 15 between the adjustment bolt 14 and the pull-rod 221, and the specific arrangement can be determined according to actual design requirements.

As shown in FIG. 15, 16, and 17, in one embodiment, the first base body 11 includes a first side plate 111, and a second side plate 112 which is arranged opposite to the first side plate 111. The first side plate 111 is provided with a first installation hole 1111, and the second side plate 112 is provided with a second installation hole 1121. The sleeve connector 12 includes a first installation end 126 and a second installation end 127, wherein the first installation end 126 penetrates through and is arranged inside the first installation hole 1111, and the second installation end 127 penetrates through and is arranged inside the second installation hole 1121. A cross-sectional shape of the first installation end 126 is non-circular, and a shape of the first installation hole 1111 is adapted to the cross-sectional shape of the first installation end 126. The first side plate 111 limits a rotation of the first installation end 126, so as to make the sleeve connector 12 non-rotatable relative to the first base body 11. In this embodiment, the limitation of the rotation of the sleeve connector 12 relative to the first base body 11 can be achieved through a structural design on the components, without the need for additional components, which has the effect of simplifying the mechanism and facilitating assembly.

It should be noted that, the rotation of the sleeve connector 12 relative to the first base body 11 can be limited in ways not limited to the above embodiments. For example, in another embodiment, a cross-sectional shape of the second installation end 127 is non-circular, a shape of the second installation hole 1121 is adapted to the cross-sectional shape of the second installation end 127. The second side plate 112 limits the rotation of the second installation end 127, so as to make the sleeve connector 12 non-rotatable relative to the first base body 11. That is to say, in this embodiment, the cross-sectional shape of the second installation end 127 is set to be non-circular, and the shape of the second installation hole 1121 can be adapted to the cross-sectional shape of the second installation end 127, so as to limit the rotation of the sleeve connector 12 relative to the first base body 11.

In another embodiment, the cross-sectional shape of the first installation end 126 and the cross-sectional shape of the second installation end 127 can both be set to be non-circular to limit the rotation of the sleeve connector 12 relative to the first base body 11.

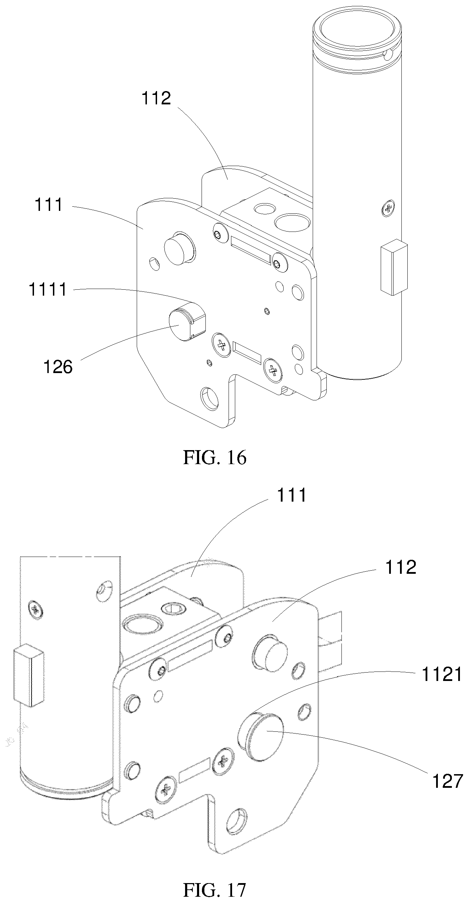

As shown in FIG. 16 to 18, in one embodiment, the sleeve 21 includes a sleeve body 212 and two first ear portions 213. The sleeve body 212 includes a first end and a second end, wherein the first end of the sleeve body 212 faces the connection base 10. Two first ear portions 213 are located at the first end of the sleeve body 212, wherein one first ear portion 213 is in rotatable connection with the first installation end 126 and the other first ear portion 213 is in rotatable connection with the second installation end 127.

In one embodiment, the first side plate 111 and the second side plate 112 are located between two first ear portions 213. Of course, not limited to the above embodiments, for example, in another embodiment, it is also possible for the two first ear portions 213 to be located between the first side plate 111 and the second side plate 112. In another embodiment, it is also possible to arrange the first side plate 111, the second side plate 112, and the two first ear portions 213 in a staggered manner, which can be determined according to actual design requirements.

As shown in FIG. 16, in one embodiment, the cross-sectional shape of the first installation end 126 is D-shaped, and the cross-sectional shape of the second installation end 127 is circular. It should be noted that the D-shape includes a planar segment and a curved surface segment. The first installation end 126 achieves a non-rotational fit with the first side plate 111 through the planar segment which is straight, and further achieves a rotational fit with the first ear portion 213 through the curved surface segment. Of course, the cross-sectional shape of the first installation end 126 is not limited to D-shape, and can also be other shapes. However, when the first installation end 126 is of other shapes, it is required that a cross-sectional profile of the first installation end 126 includes an arc segment which is greater than a half of a circular arc, or includes two opposing arc segments, or includes several spaced arc segments, so as to enable a fit between the first installation end 126 and the first installation hole 1111 to limit a rotation of the sleeve connector 12 relative to the first base body 11, and enable a rotational fit between the first installation end 126 and the first ear portion 213.

In addition, as mentioned above, the cross-sectional shape of the second installation end 127 can be non-circular, and the shape of the second installation hole 1121 can be adapted to the cross-sectional shape of the second installation end 127, so as to limit the rotation of the sleeve connector 12 relative to the first base body 11. Therefore, in another embodiment, the cross-sectional shape of the first installation end 126 and the second installation end 127 can be switched, that is, the cross-sectional shape of the first installation end 126 is circular and the cross-sectional shape of the second installation end 127 is D-shaped. Similarly, the cross-sectional shape of the second installation end 127 is not limited to D-shaped, and can also be other shapes. However, when the second installation end 127 is of other shapes, it is required that a cross-sectional profile of the second installation end 127 includes an arc segment which is greater than a half of a circular arc, or includes two opposing arc segments, or includes several spaced arc segments, so as to enable a fit between the second installation end 127 and the second installation hole 1121 to limit a rotation of the sleeve connector 12 relative to the first base body 11, and enable a rotational fit between the second installation end 127 and the first ear portion 213.

It should be noted that, as mentioned above, the sleeve connector 12 can be a shaft element as shown in FIG. 4, or a structure which is formed by the first shaft segment 122, the second shaft segment 123, and the U-shaped segment 124 as shown in FIG. 12. When the sleeve connector 12 is a shaft element as shown in FIG. 4, the first installation end 126 and the second installation end 127 are respectively the two ends of the shaft element. When the sleeve connector 12 is formed by the first shaft segment 122, the second shaft segment 123, and the U-shaped segment 124 as shown in FIG. 12, the first installation end 126 is located at an end of the first shaft segment 122, and the second installation end 127 is another end of the second shaft segment 123.

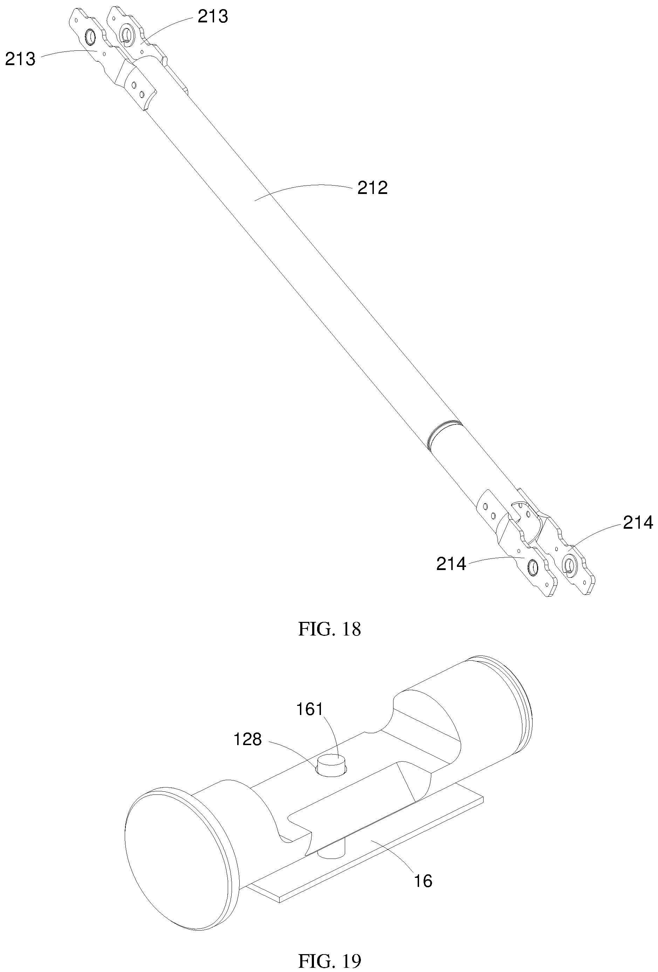

It should be noted that, the rotation of the sleeve connector 12 relative to the first base body 11 can be limited in ways not limited to the above embodiments. For example, in another embodiment, as shown in FIG. 19, the sleeve connector 12 is provided with a first limitation portion 128, and the connection base 10 further includes a limitation element 16 which is installed at the first base body 11. The limitation element 16 includes a second limitation portion 161, wherein the first limitation portion 128 and the second limitation portion 161 cooperate to limit the rotation of the sleeve connector 12 relative to the first base body 11. That is to say, in this embodiment, in order to limit the rotation of the sleeve connector 12 relative to the first base body 11, an additional limitation element 16 is added. The second limitation portion 161 of the limitation element 16 cooperates with the first limitation portion 128 of the sleeve connector 12 to limit the rotation of the sleeve connector 12 relative to the first base body 11.

In one embodiment, the first limitation portion 128 is a limitation hole which is arranged at the sleeve connector 12, and the second limitation portion 161 is a limitation column that is inserted into the limitation hole to limit the rotation of the sleeve connector 12 relative to the first base body 11. It should be noted that the positions of the limitation hole and the limitation column can be adjusted, that is, the limitation column is arranged at the sleeve connector 12, and the limitation hole is arranged at the limitation element 16, which can be determined according to actual design requirements. It should also be noted that the first limitation portion 128 and the second limitation portion 161 are not limited to a fitting method of column and hole. For example, in another embodiment, the first limitation portion 128 and the second limitation portion 161 can be set as a fitting method of protrusion and groove.

As shown in FIG. 1, in one embodiment, the support arm apparatus 100 further includes an installation base 30 and a rod element 40. A second end of the sleeve 21 is in rotatable connection with the installation base 30 about a third axis L3, and the installation base 30 is configured to connect a medical apparatus. The rod element 40 includes a first end and a second end. The first end of the rod element 40 is in rotatable connection with the connection base 10 about the fourth axis L4, and the second end of the rod element 40 is in rotatable connection with the installation base 30 about the fifth axis L5. The sleeve 21, the rod element 40, the connection base 10, and the installation base 30 are combined to form a parallelogram mechanism having a four-bar linkage. Specifically, in a plane perpendicular to the first axis L1, the sleeve 21, the rod element 40, a connection line of the first axis L1 and the fourth axis L4, and a connection line of the third axis L3 and the fifth axis L5, are combined to form a parallelogram mechanism having a four-bar linkage. In this embodiment, a parallelogram mechanism having a four-bar linkage is formed by combining the sleeve 21, the rod element 40, the connection base 10, and the installation base 30, such that according to the principle that both sides of a parallelogram remain parallel, the installation base 30 only translates but does not rotate relative to the connection base 10 during upward or downward processes, which enables the medical apparatus which is installed at the installation base 30 never to rotate relative to a horizontal axis during a height adjustment process.

In one embodiment, the rod element 40 is provided with a groove for cable management, which groove is used to accommodate cables, and with this implementation, the reasonable utilization of components can be achieved without the need for additional cable management structures, making the structure of the support arm apparatus 100 relatively compact. Moreover, by setting up the groove for cable management to manage and store cables, it is possible to avoid situations where cables are messy.

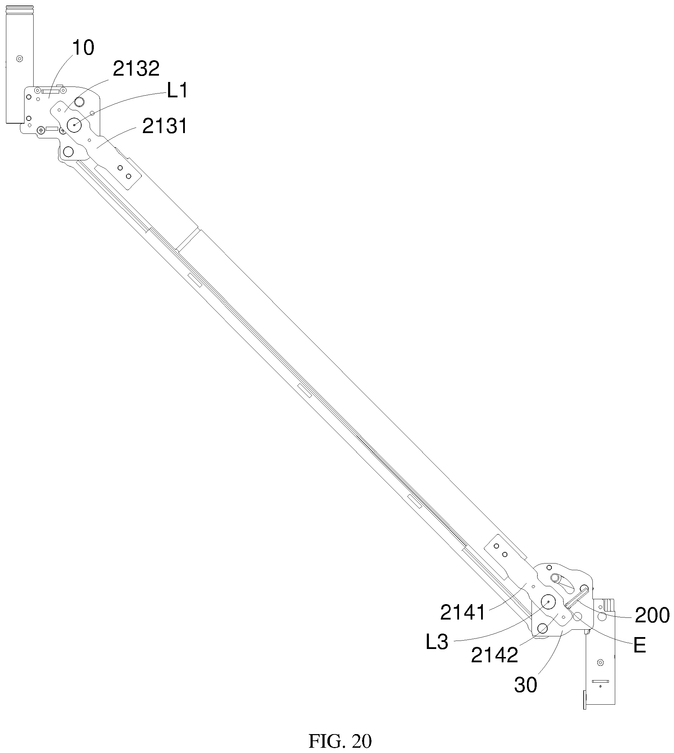

As shown in FIG. 18 and 20, in one embodiment, the first ear portion 213 is divided into a first extension segment 2131 and a second extension segment 2132 by the first axis L1, wherein the first extension segment 2131 is connected with the sleeve body 212. The sleeve 21 also includes a second ear portion 214, which is located at the second end of the sleeve body 212 and is in rotatable connection with the installation base 30 about the third axis L3. The second ear portion 214 is divided into a third extension segment 2141 and a fourth extension segment 2142 by the third axis L3, wherein the third extension segment 2141 is connected with the sleeve body 212. Wherein, the installation base 30 is provided with at least one hole E for limiting position, which is used to be inserted into a cylindrical tool 200. When the tube 20 is tilted downward relative to the connection base 10 to a predetermined angle, the hole E for limiting position is exposed. The cylindrical tool 200 is inserted into the hole E for limiting position and abuts against a top side of the fourth extension segment 2142, so as to limit an upward rotation of the tube 20 about the first axis L1. Wherein, terms "tilted downwards", "top side", and "upward rotation" are all related to an orientation of the support arm apparatus 100 in normal use.

It should be noted that the hole E for limiting position is not limited to being arranged at the installation base 30. For example, in another embodiment, the connection base 10 is provided with at least one hole E for limiting position, which is used to be inserted into a cylindrical tool 200. When the tube 20 is tilted downward relative to the connection base 10 to a predetermined angle, the hole E for limiting position is exposed. The cylindrical tool 200 is inserted into the hole E for limiting position and abuts against a bottom side of the second extension segment 2132, so as to limit an upward rotation of the tube 20 about the first axis L1. Wherein, terms "tilted downwards", "bottom side", and "upward rotation" are all related to an orientation of the support arm apparatus 100 in normal use.

Taking the application of the support arm apparatus 100 in the operating room as an example, in a usage scenario, the support arm apparatus 100 is directly or indirectly connected with a ceiling of the operating room through the connection base 10, and the installation base 30 is used to connect a medical apparatus, such as a surgical light, display, control box, and imaging head. When the medical apparatus needs to be installed at the connection base 10, or the medical apparatus installed at the installation base 30 malfunctions and needs to be repaired or replaced, the operator can pull the tube 20 to tilt and rotate downward relative to the connection base 10 to a preset angle and expose the hole E for limiting position. At this time, the operators can insert the cylindrical tool 200 carried with them, such as a screwdriver or an Allen wrench, into the hole E for limiting position. The sleeve 21 is limited by the cylindrical tool 200 and cannot rotate upwards to keep the tube 20 tilted downwards, which facilities operators to install the medical apparatus at the connection base 10 or to repair or replace the medical apparatus which is installed at the installation base 30.

The support arm apparatus 100 proposed in this embodiment is provided with a hole E for limiting position in the installation base 30 and/or the connection base 10. When the tube 20 tilts downward relative to the connection base 10 to a predetermined angle and exposes the hole E for limiting position, the operators can insert the cylindrical tool 200 carried with them into the hole E for limiting position to keep the tube 20 tilted downward. In this way, the operator can install a medical apparatus at the connection base 10 or repair or replace a medical apparatus which is installed at the installation base 30. With this implementation method, a single one operator can complete the installation of medical apparatus at the connection base 10 or repair or replace the medical apparatus installed at the installation base 30, which can save manpower and material resources.

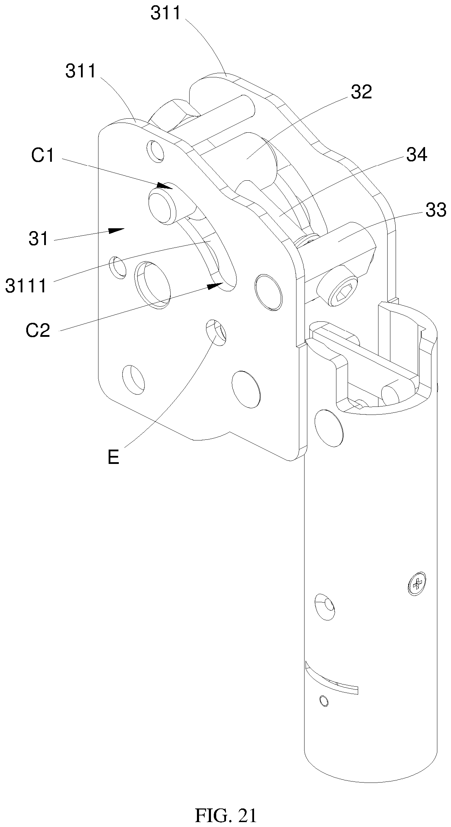

As shown in FIG. 21, in one embodiment, the installation base 30 includes a second base body 31 and a limitation element 32. The second ear portion 214 is in rotatable connection with the second base body 31, and the limitation element 32 is arranged at a top side of the second ear portion 214, and is adjustably installed at the second base body 31 along a rotation path of the fourth extension segment 2142. The limitation element 32 is used to limit the upward rotation of the fourth extension segment 2142 about the third axis L3, and the limitation hole E is located below the limitation element 32. Wherein, the limitation element 32 is used to support the arm device 100 and limit the rotation of the tube 20 under normal use. It should be noted that the "top side", "upward rotation", and "downward" mentioned are all for an orientation of the support arm apparatus 100 in normal use.

As shown in FIG. 21, in one embodiment, the second base body 31 includes two third side plates 311 which are spaced apart, and the limitation element 32 is adjustably installed at the two third side plates 311. The installation base 30 also includes an adjustment rod 33 and an adjustment bolt 34. The adjustment rod 33 is rotatably installed between two third side plates 311. The adjustment rod 33 is provided with a first screw hole or first hole along a radial direction, the limitation element 32 is provided with a second screw hole. The adjustment bolt 34 is in threaded engagement with the first screw hole or first hole and the second screw hole. By rotating the adjustment bolt 34, the limitation element 32 can be driven to implement an adjustment in a rotation path of the second extension segment 2132.

As shown in FIG. 21, in one embodiment, two third side plates 311 are provided with adjustment grooves 3111, and two ends of the limitation element 32 are respectively inserted into the adjustment grooves 3111 of the two third side plates 311 and adjustable along the adjustment grooves 3111. When it is necessary to adjust the position of the limitation element 32, the operator rotates the adjustment bolt 34, which drives the limitation element 32 to move along the adjustment groove 3111.

In one embodiment, one end of the adjustment bolt 34 is provided with a sink groove. The sink groove can be, but is not limited to, a straight sink groove, a cross sink groove, or a hexagonal sink groove. In this way, when the adjustment bolt 34 needs to be rotated, the operator can insert a straight screwdriver, a cross screwdriver or a hexagonal wrench into the sink groove and rotate it to drive the adjustment bolt 34 to rotate.

As shown in FIG. 21, in one embodiment, the adjustment groove 3111 includes a first end C1 and a second end C2, wherein the first end C1 is higher than the second end C2, and a height of the hole E for limiting position is lower than that of second end C2.

As shown in FIG. 21, in one embodiment, the adjustment groove 3111 is an arc with the third axis L3 as a center of circle. Of course, the adjustment groove 3111 is not limited to an arc, but can also be a straight line or other shape, which can be determined according to actual design requirements.

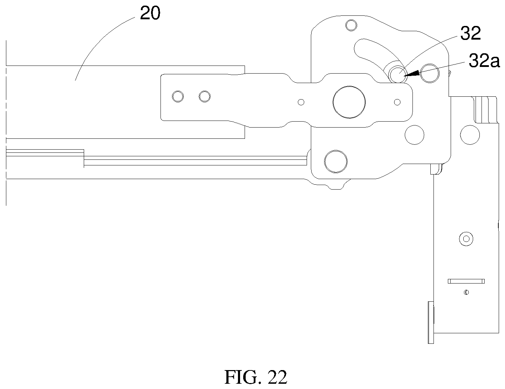

As shown in FIG. 22, in one embodiment, the limitation element 32 includes a first position 12a which is closest to the limitation hole E. When the limitation element 32 is located at the first position 12a, the tube 20 remains horizontal under the limitation of the limitation element 32. Of course, the tube 20 may not necessarily remain horizontal under the limitation of the limitation element 32, and may also have a certain angle relative to a horizontal plane, which can be determined according to actual design requirements.

As shown in FIG. 1 to 17, an embodiment of this disclosure also proposes a support arm apparatus 100 which is configured to support a medical apparatus. The proposed support arm apparatus 100 includes a connection base 10, and a tube 20 which includes a spring. The connection base 10 includes a first base body 11, a sleeve connector 12, and a pull-rod connector 13, wherein the sleeve connector 12 and the pull-rod connector 13 are installed at the first base body 11. The tube 20 includes a sleeve 21 and an elastic part 22. The sleeve 21 includes a first end and a second end, wherein the first end of the sleeve 21 is in rotatable connection with the sleeve connector 12 about a first axis L1. The elastic part 22 includes a pull-rod 221 and a spring-rod part 222. The pull-rod 221 includes a first end and a second end. The first end of the pull-rod 221 is in rotatable connection with the pull-rod connector 13 about a second axis L2. The second end of the pull-rod 221 is in rotatable connection with the spring-rod part 222, which is installed inside the sleeve 21. The spring-rod part 222 is configured to provide a balance force to enable the support arm apparatus 100 to support a weight of the medical apparatus. The sleeve connector 12 is located in a rotation path of the pull-rod 221. The sleeve connector 12 is provided with an avoidance structure A. The avoidance structure A is capable of avoiding at least a portion of the pull-rod 221, so as to enlarge a range of rotation of the pull-rod 211 towards the sleeve connector 12, during said rotation of the pull-rod 211 towards the sleeve connector 12. Optionally, the avoidance structure A includes a second groove 125 as shown in FIG. 8.

The support arm apparatus 100 proposed in this embodiment provides a second groove 125 at the sleeve connector 12 for avoiding the pull-rod 221, so that the pull-rod 221 has a larger angle of rotation towards the sleeve connector 12, that is, the tube 20 has a larger depression angle of rotation, which can facilitate adjustment, installation, and maintenance of medical apparatus by users.

As shown in FIG. 4, in one embodiment, the sleeve connector 12 is a shaft element, and the avoidance structure A includes a groove which is arranged at the sleeve connector 12. During a rotation of the pull-rod 221 towards the sleeve connector 12, at least a portion of the pull-rod 221 is capable of being accommodated inside the groove.

As shown in FIG. 10, in one embodiment, the sleeve connector 12 is a shaft element, and the avoidance structure A includes a side plane A1 which is arranged at the sleeve connector 12 and parallel to the first axis L1. The side plane A1 extends from a first end of the shaft element to a second end of the shaft element. During a rotation of the pull-rod 221 towards the sleeve connector 12, at least a portion of the pull-rod 221 is capable of being avoided by the side plane A1.

As shown in FIG. 12, in one embodiment, the sleeve connector 12 includes a first shaft segment 122, a second shaft segment 123, and a U-shaped segment 124. The first shaft segment 122 and the second shaft segment 123 are connected with the first base body 11. The U-shaped segment 124 is connected between the first shaft segment 122 and the second shaft segment 123. A space which is enclosed by the U-shaped segment 124 forms the avoidance structure A. Optional, the avoidance structure A can be formed by the U-shaped segment 124, the first shaft segment 122 and the second shaft segment 123 together.

As shown in FIG. 14, in one embodiment, a convex portion 2211 is arranged at a side of the pull-rod 221, which side faces the sleeve connector 12. When the pull-rod 221 is rotated to an extreme position of the first range of rotation, which position faces the sleeve connector 12, the convex portion 2211 is capable of avoiding at least a portion of the sleeve connector 12 to further enlarge the range of rotation of the pull-rod 221 toward the sleeve connector 12.

In one embodiment, the pull-rod 221 includes a fitting segment 2212, which is bent away from the sleeve connector 12 to form a convex portion 2211. The bent shape of the fitting segment 2212 can be an arc shape, a curved shape, a broken line shape, etc.

The other structures, connection relationships, and beneficial effects of the support arm apparatus 100 proposed in this embodiment can refer to the above embodiments and are not repeated here.

The embodiments of this disclosure also propose a medical device including a medical apparatus and the aforementioned support arm apparatus 100, wherein the medical apparatus is installed at the second end of the sleeve 21. Optionally, the medical device is at least one of a surgical light, a display, a control box, and an imaging head.

The above are only specific embodiments of this disclosure; the scope of protection of this disclosure is not limited to this. Any skilled person familiar with the technical field can easily think of various equivalent modifications or substitutions within the technical scope disclosed in this disclosure, and these modifications or substitutions should be included in the scope of protection of this disclosure. Therefore, the scope of protection of this disclosure should be based on the scope of protection of the claims.

## Claims

1. A support arm apparatus which is configured to support a medical apparatus, **characterized in that**, comprising a connection base and a tube comprising a spring; wherein the connection base comprises a first base body, a sleeve connector, and a pull-rod connector;
the sleeve connector is installed at the first base body, the pull-rod connector is adjustably installed at the first base body, and is capable of moving towards and away from the sleeve connector during adjustment;
the tube further comprises:
a sleeve, which comprises a first end and a second end, wherein the first end of the sleeve is in rotatable connection with the sleeve connector about a first axis; and
an elastic part, which comprises a pull-rod and a spring-rod part, wherein the pull-rod comprises a first end and a second end, the first end of the pull-rod is in rotatable connection with the pull-rod connector about a second axis, the second end of the pull-rod is in rotatable connection with the spring-rod part, wherein the spring-rod part is installed inside the sleeve, and configured to provide a balance force to enable the support arm apparatus to support a weight of the medical apparatus;
wherein, the sleeve connector is located in a rotation path of the pull-rod;
the sleeve connector is provided with an avoidance structure, wherein the avoidance structure is capable of avoiding at least a portion of the pull-rod connector, so as to enlarge a range of movement of the pull-rod connector towards the sleeve connector; and/or the avoidance structure is capable of avoiding at least a portion of the pull-rod, so as to enlarge a range of rotation of the pull-rod towards the sleeve connector.

2. The support arm apparatus according to claim 1, **characterized in that**, the sleeve connector is a shaft element, the avoidance structure comprises a first groove which is arranged at the sleeve connector;
when the pull-rod connector is moved to one extreme position which faces the sleeve connector, at least a portion of the pull-rod connector is capable of being accommodated inside the first groove; and/or
when the pull-rod is rotated to another extreme position which faces the sleeve connector, at least a portion of the pull-rod is capable of being accommodated inside the first groove.

3. The support arm apparatus according to claim 2, **characterized in that**, the first groove comprises a first bottom wall, and two first side walls which are respectively arranged at both ends of the first bottom wall; wherein the first bottom wall is parallel to the first axis, the two first side walls are spaced apart along the first axis;
a distance between the two first side walls is not less than a width of a corresponding portion of the pull-rod connector; wherein when the pull-rod connector is moved to the one extreme position which faces the sleeve connector, at least a portion of the pull-rod connector is capable of entering a space which is defined by the first bottom wall and the two first side walls; and/or
a distance between the two first side walls is not less than a width of a corresponding portion of the pull-rod; wherein when the pull-rod is rotated to the another extreme position which faces the sleeve connector, at least a portion of the pull-rod is capable of entering a space which is defined by the first bottom wall and the two first side walls.

4. The support arm apparatus according to claim 3, **characterized in that**, the avoidance structure further comprises a second groove which is arranged at the sleeve connector; wherein the second groove comprises a second bottom wall, and two second side walls which are respectively arranged at both ends of the second bottom wall, wherein the second bottom wall is parallel to the first axis, the second bottom wall intersects with the first bottom wall to form an obtuse angle therebetween; the two second side walls are spaced apart along the first axis, a distance between the two second side walls is not less than a width of a corresponding portion of the pull-rod;
wherein when the pull-rod is rotated to the another extreme position which faces the sleeve connector, at least a portion of the pull-rod is capable of entering a space which is defined by the second bottom wall and the two second side walls; and/or
wherein when the pull-rod connector is moved to the one extreme position which faces the sleeve connector, at least a portion of the pull-rod connector is capable of entering a space which is defined by the second bottom wall and the two second side walls.

5. The support arm apparatus according to claim 3 or claim 4, **characterized in that**, the pull-rod connector is moved along a first direction towards and away from the sleeve connector during adjustment, wherein the first direction is perpendicular to the first bottom wall.

6. The support arm apparatus according to claim 1, **characterized in that**, the sleeve connector is a shaft element, and the avoidance structure comprises a side plane which is arranged at the sleeve connector; wherein the side plane is configured to avoid at least a portion of the pull-rod connector during a movement of the pull-rod connector towards the sleeve connector; and/or the side plane is configured to avoid at least a portion of the pull-rod during a rotation of the pull-rod towards the sleeve connector.

7. The support arm apparatus according to claim 6, **characterized in that**, the shaft element is a portion of a cylinder, and the shaft element is cut by a plane, which plane is parallel to the first axis, so as to form the side plane, wherein the side plane extends from a first end of the shaft element to a second end of the shaft element, a cross-section of the cylinder is circular, and a corresponding central angle of the side plane on the cross-section is less than 180 degrees.

8. The support arm apparatus according to claim 1, **characterized in that**, the sleeve connector comprises a first shaft segment, a second shaft segment, and a U-shaped segment, wherein the first shaft segment and the second shaft segment are connected with the first base body, the U-shaped segment is connected between the first shaft segment and the second shaft segment; the avoidance structure is formed by the U-shaped segment; or the avoidance structure is formed by the U-shaped segment, the first shaft segment and the second shaft segment together;
when the pull-rod connector is moved to one extreme position which faces the sleeve connector, at least a portion of the pull-rod connector is capable of being accommodated by the avoidance structure; and/or
when the pull-rod is rotated to another extreme position which faces the sleeve connector, at least a portion of the pull-rod is capable of being accommodated by the avoidance structure.

9. The support arm apparatus according to claim 8, **characterized in that**, the U-shaped segment comprises a portion which is parallel to the first axis, wherein a third groove is arranged at said portion which is parallel to the first axis; when the pull-rod is rotated to another extreme position of a first range of rotation, which position faces the sleeve connector, at least a portion of the pull-rod is capable of being accommodated inside the third groove.

10. The support arm apparatus according to claim 1, **characterized in that**, the sleeve connector is non-rotatably installed at the first base body; the sleeve connector comprises a first installation end and a second installation end which ends penetrate through and are arranged inside the first base body; a cross-sectional shape of at least one of the first installation end and the second installation end is non-circular, and comprises an arc segment which is in a rotational fit with the sleeve.

11. A support arm apparatus which is configured to support a medical apparatus, **characterized in that**, comprising a connection base and a tube comprising a spring; wherein the connection base comprises a first base body, a sleeve connector, and a pull-rod connector; wherein the sleeve connector and the pull-rod connector are installed at the first base body;
the tube comprises:
a sleeve, which comprises a first end and a second end, wherein the first end of the sleeve is in rotatable connection with the sleeve connector about a first axis; and
an elastic part, which comprises a pull-rod and a spring-rod part, wherein the pull-rod comprises a first end and a second end, the first end of the pull-rod is in rotatable connection with the pull-rod connector about a second axis, the second end of the pull-rod is in rotatable connection with the spring-rod part, wherein the spring-rod part is installed inside the sleeve, and configured to provide a balance force to enable the support arm apparatus to support a weight of the medical apparatus;
wherein, the sleeve connector is located in a rotation path of the pull-rod;
the sleeve connector is provided with an avoidance structure; the avoidance structure is capable of avoiding at least a portion of the pull-rod, so as to enlarge a range of rotation of the pull-rod towards the sleeve connector, during said rotation of the pull-rod towards the sleeve connector.

12. The support arm apparatus according to claim 11, **characterized in that**, the sleeve connector is a shaft element, the avoidance structure comprises a groove which is arranged at the sleeve connector;
at least a portion of the pull-rod connector is capable of being accommodated inside the groove, during said rotation of the pull-rod towards the sleeve connector.

13. The support arm apparatus according to claim 11, **characterized in that**, the sleeve connector is a shaft element, the avoidance structure comprises a side plane which is arranged at the sleeve connector and parallel to the first axis, wherein the side plane extends from a first end of the shaft element to a second end of the shaft element; wherein the side plane is configured to avoid at least a portion of the pull-rod connector, during said rotation of the pull-rod towards the sleeve connector.

14. The support arm apparatus according to claim 11, **characterized in that**, the sleeve connector comprises a first shaft segment, a second shaft segment, and a U-shaped segment, wherein the first shaft segment and the second shaft segment are connected with the first base body, the U-shaped segment is connected between the first shaft segment and the second shaft segment; the avoidance structure is formed by the U-shaped segment; or the avoidance structure is formed by the U-shaped segment, the first shaft segment and the second shaft segment together.

15. A medical device, **characterized in that**, comprising:
a medical apparatus; and
the support arm apparatus according to any one of claims 1 to 14;
wherein the medical apparatus is installed at the second end of the sleeve.
